# EUROPEAN PATENT APPLICATION

(11) **EP 3 223 179 A1**
(43) Date of publication of application: **27.09.2017**
(21) Application number: 16201280.1
(22) Date of filing: 29.11.2016
(51) Int. Cl.: G06F 19/00, G06F 17/30

(54) **A HEALTHCARE RISK EXTRACTION SYSTEM AND METHOD**

(30) Priority: 24.03.2016 GB 201605113; 24.03.2016 DE 102016205065
(71) Applicant: FUJITSU LIMITED, Kawasaki-shi Kanagawa 211-8588 (JP)
(72) Inventor: DE LA TORRE, Victor, 28007 Madrid (ES); VILLAZON-TERRAZAS, Boris, 28003 Madrid (ES)
(74) Representative: Wilding, Frances Ward

(57) **Abstract**

A healthcare risks extraction system comprising:
a risk related terms collector to accept input of terms by a clinician, the clinician's terms including terms related to risks in the form of potential diseases, terms related to risk factors that increase the likelihood of disease and terms related to treatments of a medical condition;
a medical entity reconciliator, to standardise and expand the clinicians' terms to include synonyms and equivalent terms using a standardised vocabulary of terms;
a topic detector and tagger, to retrieve a set of documents linked to the expanded terms from a medical document database;
a named entity recognition, resolution and disambiguation, NERD, module to extract entities from the set of document and each aligned to the standardised vocabulary; and
a relation extractor to score relations between the entities based on the co-occurrence of two entities in documents in the retrieved set of documents; wherein
the healthcare risks extraction system is arranged to generate a risk knowledge graph storing the entities and their scored relations.

## Description

The present invention relates to assessing healthcare of an individual or subject, usually referred to as a patient. The patient may be a human or potentially an animal, such as a specimen of a rare breed or even a pet. In many scenarios, the patient may already be suffering from a disorder, but in others the patient is currently healthy. The invention is thus widely applicable in medicine, healthcare and veterinary science.

In medicine, risk can be seen as the probability of a negative outcome on the health of a patient or on a population of patients. Health risk factors can be viewed as attributes, characteristics or exposures that increase the likelihood of a person developing a disease or health disorder.

One of the important tasks in medicine is the assessment of risks. This task may rely on scientific knowledge derived from rigorous medical studies that identify factors impacting clinical changes, along with their quantification of those factors. However, current risk assessment solutions usually only take into account very limited medical knowledge for the risk evaluation, and in most of the cases this risk related knowledge is hardcoded within the solution.

In clinical practice, many protocols have been designed to estimate the risk of a patient developing different conditions. However in most cases the health risks for a given patient are represented as a plain list, whereas the truth is that these risks are interconnected. The links between the different risks can be established at different levels (that is with different forms and/or with different weights). For example risks included in the genetic background of the patient, the adverse effects of the medicines, the life style, etc are all different links.

Lately, the health research community has been making good progress in collecting and providing access to useful health data such as genomic, toxicology, exposure, and disease data. A particular obstacle to applying these volumes of data in the field of risk assessment is the lack of methods, tools, and techniques to collect, clean, and process millions of journal publications, hundreds of databases, and dozens of ontologies to discover relations among exposure, drugs, treatments, and diseases.

According to an embodiment of a first aspect of the invention, there is provided a healthcare risks extraction system comprising:
a risk related terms collector to accept input of terms by a clinician, the clinician's terms including terms related to risks in the form of potential diseases, terms related to risk factors that increase the likelihood of disease and terms related to treatments of a medical condition;
a medical entity reconciliator, to standardise and expand the clinicians' terms to include synonyms and equivalent terms using a standardised vocabulary of terms;
a topic detector and tagger, to retrieve a set of documents linked to the expanded terms from a medical document database;
a named entity recognition, resolution and disambiguation, NERD, module to extract entities from the set of document each with a score and each aligned to the standardised vocabulary; and
a relation extractor to score relations between the entities based on the co-occurrence of two entities in documents, and potentially also on the context in the retrieved set of documents; wherein
the healthcare risks extraction system is arranged to generate a risk knowledge graph storing the entities and their scored relations.

The risk knowledge graph blends clinician knowledge (from one or more clinicians) with open data to provide a new set of information which is invaluable to the user in presenting risks and their relation to risk factors and treatments.

The system may further comprises a knowledge graph curator, to display the risk knowledge graph and to accept clinician input to manually curate the generated graph.

The risk related terms collector may be arranged to accept the terms as a list (or lists) of terms per category of risk, risk factor and treatment. This can be by input of plain text, and the clinician (or clinicians) does not need to enter any other information, such as links between the terms.

The topic detector (and tagger) can be arranged to take into account the provenance of the documents, for example which journal they came from, the journal date etc. This provenance can be taken into account potentially for scoring and other purposes later.

In this case, the risk knowledge graph can also store the provenance of the entities. This can provide that extra information to the user.

The risk related terms collector (or another component of the system) may be arranged to accept annotations by the clinician of the standardised vocabulary of terms, the annotations labelling vocabulary in categories of risks, risk factors and treatments.

The topic detector and tagger may be arranged to tag the documents according to categories of risks, risk factors and treatments and additionally according to the main topic of the document, which is not necessarily a risk, risk factor or treatment. This information may be available due to the annotations entered as explained above. This tagging process is important because it can identify the main topic of the documents, and then the system can create relations between this primary topic and the named entities of the document. This is one particular way to deal with the context.

In some embodiments, the NERD module scores each entity to reflect the accuracy of a match between the standardised vocabulary term and the corresponding term or terms in the retrieved linked documents.

The system may further comprise a user input to accept input of terms by a user and/or a subgraph selection module to select a relevant part of the graph for display to the user. For example, this functionality may be provided using a GUI, Graphical User Interface.

The system may further comprise a translation module to accept a term in one language and translate it into the equivalent in the language of the standardised vocabulary.

According to an embodiment of a further aspect of the invention there is provided a computer-implemented healthcare risks extraction method comprising:
accepting input of terms by a clinician, the clinician's terms including terms related to risks in the form of potential diseases, terms related to risk factors that increase the likelihood of disease and terms related to treatments of a medical condition;
standardising and expanding the clinicians' terms to include synonyms and equivalent terms using a standardised vocabulary of terms;
retrieving a set of documents linked to the expanded terms from a medical document database;
extracting entities from the set of document each aligned to the standardised vocabulary;
scoring relations between the entities based on the co-occurrence of two entities in documents, and optionally on the context in the retrieved set of documents; wherein a risk knowledge graph storing the entities and their scored relations is generated.

According to an embodiment of a further aspect of the invention there is provided a computer program which when executed on a computer carries out a method according as defined above.

A method or computer program according to preferred embodiments of the present invention can comprise any combination of the previous apparatus aspects, but without restriction as to the specific parts of the system involved. Methods or computer programs according to these further embodiments can be described as computer-implemented in that they require processing and memory capability.

The apparatus according to preferred embodiments is described as configured or arranged to, or simply "to" carry out certain functions. This configuration or arrangement could be by use of hardware or middleware or any other suitable system. In preferred embodiments, the configuration or arrangement is by software.

Thus according to one aspect there is provided a program which, when loaded onto at least one computer configures the computer to become the system according to any of the preceding system definitions or any combination thereof.

According to a further aspect there is provided a program which when loaded onto the at least one computer configures the at least one computer to carry out the method steps according to any of the preceding method definitions or any combination thereof.

In general the computer may comprise the elements listed as being configured or arranged to provide the functions defined. For example this computer may include memory, processing, and a network interface.

The invention can be implemented in digital electronic circuitry, or in computer hardware, firmware, software, or in combinations of them. The invention can be implemented as a computer program or computer program product, i.e., a computer program tangibly embodied in a non-transitory information carrier, e.g., in a machine-readable storage device, or in a propagated signal, for execution by, or to control the operation of, one or more hardware modules. A computer program can be in the form of a stand-alone program, a computer program portion or more than one computer program and can be written in any form of programming language, including compiled or interpreted languages, and it can be deployed in any form, including as a stand-alone program or as a module, component, subroutine, or other unit suitable for use in a data processing environment. A computer program can be deployed to be executed on one module or on multiple modules at one site or distributed across multiple sites and interconnected by a communication network.

Method steps of the invention can be performed by one or more programmable processors executing a computer program to perform functions of the invention by operating on input data and generating output. Apparatus of the invention can be implemented as programmed hardware or as special purpose logic circuitry, including, e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention can be performed in a different order and still achieve desirable results. Multiple test script versions can be edited and invoked as a unit without using object-oriented programming technology; for example, the elements of a script object can be organized in a structured database or a file system, and the operations described as being performed by the script object can be performed by a test control program.

Elements of the invention have been described using the terms "module" and "unit" and functional definitions. The skilled person will appreciate that such terms and their equivalents may refer to parts of the system that are spatially separate but combine to serve the function defined. Equally, the same physical parts of the system may provide two or more of the functions defined.

For example, separately defined means may be implemented using the same memory and/or processor as appropriate.

Preferred features of the present invention will now be described, purely by way of example, with references to the accompanying drawings, in which:-
Figure 1 is a block diagram of components in a general embodiment of the invention;
Figure 2 is a flow chart of a method in a general embodiment;
Figure 3 is a block diagram of the main system components in a detailed embodiment;
Figure 4 is a basic example of medical entity reconciliation;
Figure 5 is a block diagram illustrating topic tagging and detection of PUBMED documents;
Figure 6 is a block diagram illustrating named entity resolution and disambiguation;
Figure 7 is a block diagram illustrating relation extraction;
Figure 8 is an illustration of an excerpt from a knowledge graph; and
Figure 9 is a diagram of suitable hardware for implementation of invention embodiments.

In summary, the inventors have come to the realisation that, within the health domain:
- there are no standards for representing health risks, along with their risk factors, and their relationships to drugs, treatments and diseases,
- there is a lack of methods and tools to extract, curate, reconcile and integrate data about risk, and risk factors.

Therefore, invention embodiments can aim:
- To identify the related concepts to risk and risk factors, and potentially validate those definitions with clinicians.
- To create a graph of Health Risks along with risk factors, and their relationships to drugs, treatments, and diseases. The data can be extracted from literature, and public data sources, together with the clinicians' expertise on risk assessment.

Precision medicine is a medical model that proposes the customisation of healthcare, tailored to the individual patient/subject. This is an emerging approach for disease diagnosis, treatment and prevention that takes into account individual variability in genes, physiology, anatomy, environment, and lifestyle. In this context invention embodiments aim to create a Knowledge Graph of health risks along with their risk factors, and their associated treatments, diagnosis, and drugs.

The following definitions are used in this document:
Health risk: a disease precursor associated with a higher than average morbidity or mortality rate. Disease precursors include demographic variables, certain individual behaviours, familial and individual histories, and certain physiological changes.
Health risk factor: a condition, behaviour, or other factor that increases risk, e.g., depression is a risk factor in suicide.

Medical treatment: the management and care of a patient, including for example in the mental health area, nursing, psychological intervention and specialist mental health rehabilitation. This term may also include "alternative" medical treatments and medication which may be prescribed, if so wished, for example, homeopathic/hypnosis/ acupuncture treatment.
Diagnosis: the process of determining by examination the nature and circumstance of a disease or condition from its signs and symptoms
Drugs: medicaments that treat or prevent or alleviate the symptoms of a disease or condition.

As far as the inventors are aware, there is no standard resource for dealing with health risks, there are only ad-hoc resources such as plain lists, or matrices within medical institutions and for specific areas.

In summary:
- there are no standards for representing health risks, in the same way as there are standards for diseases, e.g., ICD9 and ICD10 (The ninth and tenth revisions of the International Classification of Diseases); there are only plain lists of risks and they are specific to a particular medical institution or area;
- there is a lack of methods and tools to extract, curate, reconcile and integrate data about risk, and risk factors, from medical journal publications, databases, and ontologies.

Invention embodiments create a Knowledge Graph of medical risks along with their risk factors and their relations to diseases, treatments, drugs, and symptoms.

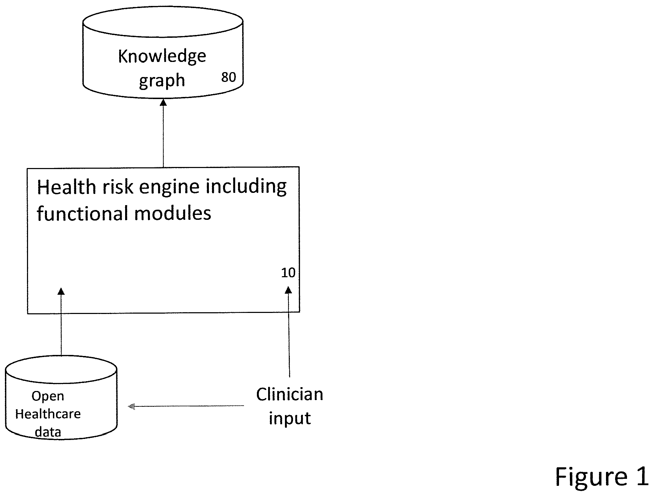

Figure 1 shows a healthcare risks extraction system according to a general embodiment, it essentially comprises health risk engine 10, which accepts inputs from clinicians and is connected to open data in the form of a standardised vocabulary of terms and a library of documents from the healthcare domain, nursing, veterinary, healthcare, medical and scientific articles. Individual modules are explained further below, but are not represented within the health risk engine, for simplicity.

A risk related terms collector accepts input of terms by a clinician (or from a group of clinicians). These clinician's terms including terms related to risks in the form of potential diseases, terms related to risk factors that increase the likelihood of disease and terms related to treatments of a medical condition.

A medical entity reconciliator is used to standardise and expand the clinicians' terms to include synonyms and equivalent terms using a standardised vocabulary of terms. For example the SNOMED ontologies may be used, as explained in more detail later.

A topic detector is used to retrieve a set of documents linked to the expanded terms from a searchable medical document database (such as PUBMED). Essentially, this component compares the documents contents (for example their abstracts) with the standardised terms and selects the documents which include exactly those terms or close matches to those terms.

A named entity recognition, resolution and disambiguation, NERD, module extracts entities from the set of document each with a score and each aligned to the standardised vocabulary. That is, the entity may be taken from the SNOMED vocabulary, for example, but is matched to the document content.

A relation extractor scores relations between the entities based on the co-occurrence of two entities in documents, and on the context in the retrieved set of documents. For example, this can use known co-occurrence metrics and any other appropriate techniques.

The healthcare risks extraction system is arranged to generate a risk knowledge graph 80 storing the entities and their scored relations. The graph may be generated by the parts explained above. The graph can then be displayed to the user (who might for instance be another clinician). For example the user might enter a term, such as a risk, risk factor or treatment and receive a subgraph of the linked terms and the strength of the link, based on the knowledge implicitly stored in the PUBMED library.

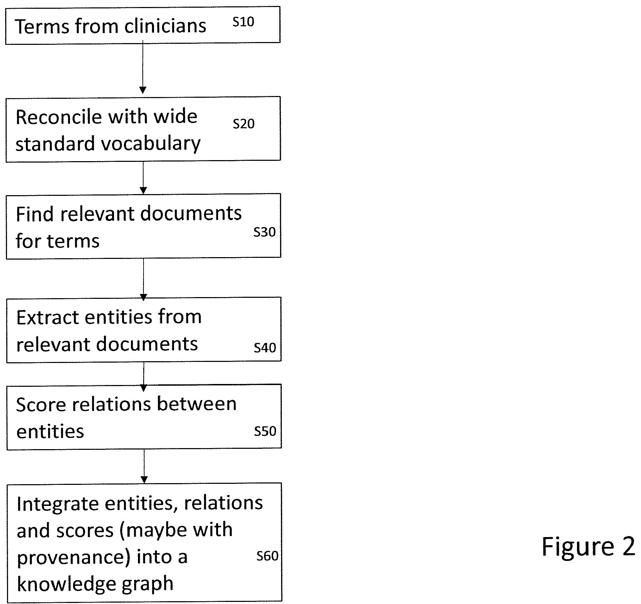

Figure 2 shows a corresponding flowchart. In S10 the system accepts input of terms by a clinician. In step S20, these are reconciled with a wide standard vocabulary to expand and standardise them. In step S30 the relevant documents are retrieved from a document database, as those linked to the expanded terms. In step S40, entities are extracted from the set of document, each entity being aligned to the standardised vocabulary., In step S50, relations between the entities are scored based on the co-occurrence of two (or more) entities in documents in the retrieved set of documents; and finally in step S60 a risk knowledge graph is generated storing the entities and their scored relations. The provenance of the entities may also be stored, for example in the form of document IDs(not the whole documents). As mentioned above, the previous steps may together provide the graph, and in this case a separate step S60 is not required.

A detailed embodiment might consist of the following main modules
- A module for collecting a set of risk related terms from the clinicians' knowledge.
- A module that reconciles the risk related terms to SNOMED (Systemized Nomenclature of Medicine) entities.
- A module that generates an initial knowledge model from the set of risk related definitions and links to SNOMED concepts.
- A module that performs risk topic document identification over PUBMED documents based on the identified SNOMED concepts. PUBMED is a service of the US National Library of Medicine (NLM) and provides free access to the NLM database of nursing, veterinary, healthcare, medical and scientific articles
- A module for performing, named entity recognition, resolution and disambiguation (NERD) over the resultant documents from the previous module.
- A module for performing relation extraction over the extracted entities from the previous module
- A module that curates, and refines the entities and relations with the support of the clinicians.

One underlying concept is that the data used covers a wide range of different risks and risk factors: invention embodiments are not limited to a certain area of medicine. For example SNOMED CT (clinical terms) is a standardised multilingual vocabulary which is generally applicable across medical and health care areas. DUBMED is also as wide-ranging as the US NLM and thus generally applicable.

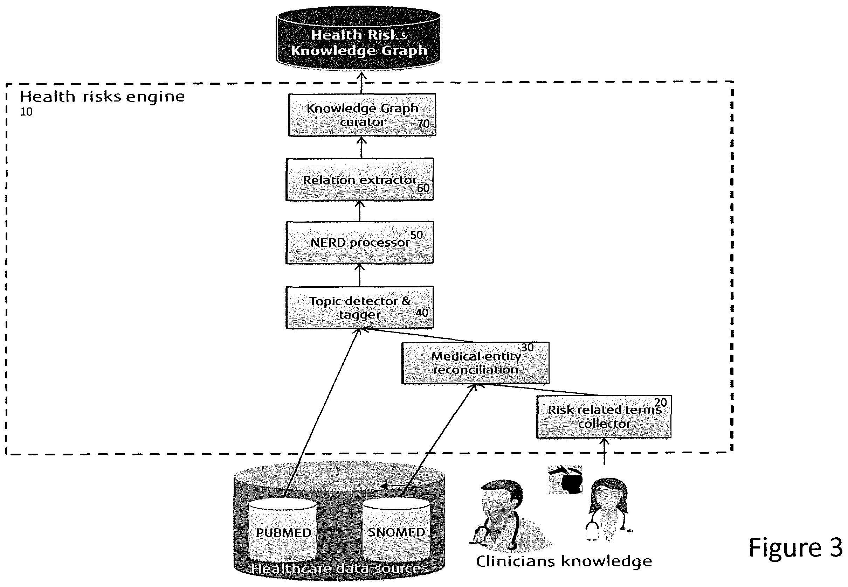

In a nutshell the system, or health risk engine 10, of one detailed embodiment comprises six main modules that are described in the following and are depicted in Figure 3.

### Risk related terms collector 20

This component is in charge of interacting with one or more healthcare practitioners, doctors, nurses, and veterinary practitioners etc, hereinafter referred to as "clinicians" who inputs the seed of risk related terms into the system. According to the clinicians the terms will be grouped in three main groups
- terms related to risk,
- terms related to risk factors; and
- terms related to treatments.

According to the definition of risk, the health risk is the probability of a negative outcome on the health of a patient, in which a negative outcome may be a particular disease or even death. Therefore, the terms within the risk group are going to be a list of potential diseases (or conditions, usually including illness or disorder).

The terms are entered subdivided into the groups below by the clinicians.

Risk factors are grouped, for example in the following sub-groups
- environmental
- demographic/environmental
   o age
   o sex
   o race
   o location
   o religion
- genetic, tendency to conditions hardcoded in the human genome
- behavioural, as examples we can have
   o tobacco smoking
   o unhealthy eating
   o physical inactivity
   o alcohol consumption
   o unsafe sex
- biomedical, that may include clinical diagnoses and states, which can influence patient health
   o diabetes
   o pregnancy
   o high blood pressure
   o impaired fasting glucose

Finally, **treatments** can be grouped in the following subgroups
- drugs, including administration method and dosage
- surgical procedure
- administration scheme, which has frequency and duration

It is worth mentioning that this is a tentative an initial set of terms suggested by the clinician expertise, it is not an exhaustive or complete list at this stage.

The component will collect and store the enhanced set of terms into the system.

### Medical entity reconciliation 30

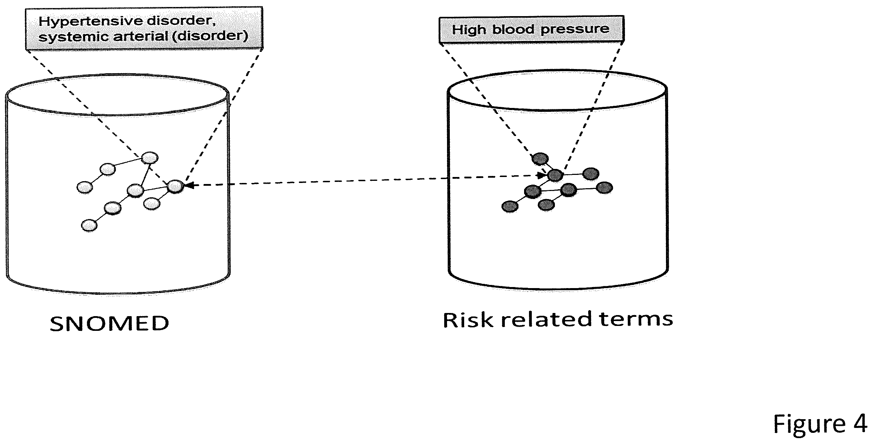

This component aims at identifying multiple representation of the same real-word object, in other words identifying equivalent terms in the two different data sources. In this particular case by performing matching/alignment between the collected terms from the clinicians and SNOMED, a standardised multilingual vocabulary of terms related to the care of the individual. The outcome of the component is to have the enhanced set of terms, proposed by the clinicians, annotated in terms of SNOMED. For example *high blood pressure* is a term, coming from the suggested risk terms by the clinicians, that corresponds to the *Hypertensive disorder, systemic arterial (disorder)* from SNOMED. The reconciliation will adopt the SNOMED and put the clinician's term as a potential synonym. This process can rely on existing, available, approaches for aligning terms from two different sources. Figure 4 illustrates the example provided previously.

### Topic detection and tagging processor 40

Once we have reconciled the input information in terms of SNOMED vocabulary, it is time to extract the set of documents of PUBMED and perform topic detection and tagging of them according to the SNOMED terms. Basically, this component will detect and tag the related categories we have identified before
- Risks, along with the descriptions that it includes
- Risk factors, including the items identified by the clinicians
- Treatments, along with the identified sub-categories

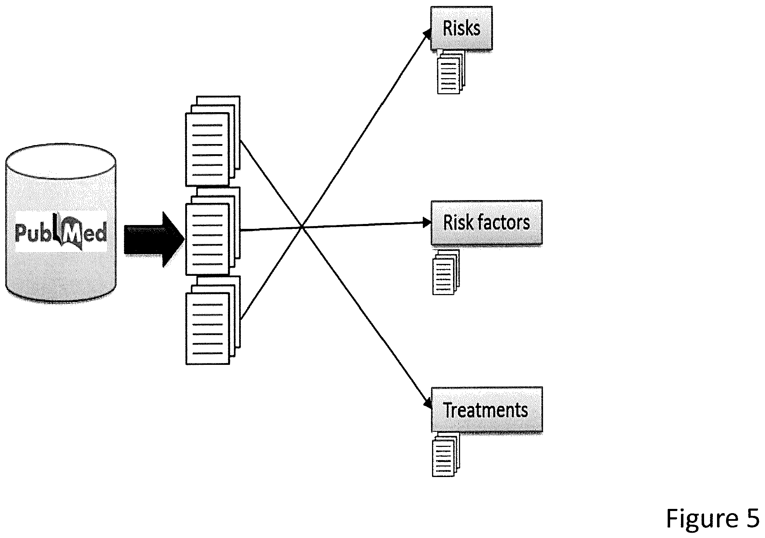

The output of the component will be a cluster of PUBMED documents group by each one of these categories. The document can be included in one or more categories. Figure 5 depicts the general flow of this component.

### NERD processor 50

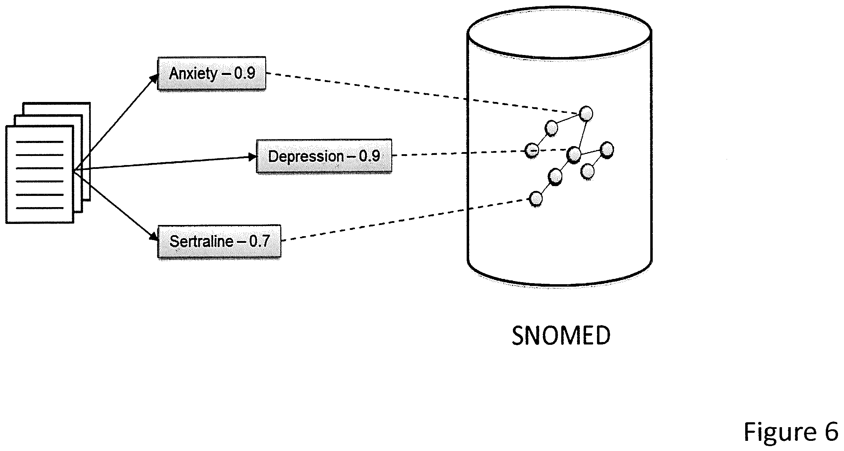

This component is in charge of recognizing and disambiguating the medical entities from each cluster of PUBMED documents previously generated. The output of the component is a set of extracted entities, along with their scores, aligned to SNOMED concepts. Figure 6 depicts the flow of the component. This component will reuse some of the functionalities of the medical entity reconciliation component.

### Relation extractor 60

The main goal of this component is to extract the relations of the previously identified entities. The extracted relations will also have a score based on the number of publications in which the relation was present. This can rely on existing, available approaches for co-occurrence scoring based, for example, on the number of documents which contain both entities divided by the total number of documents; and based on the context.

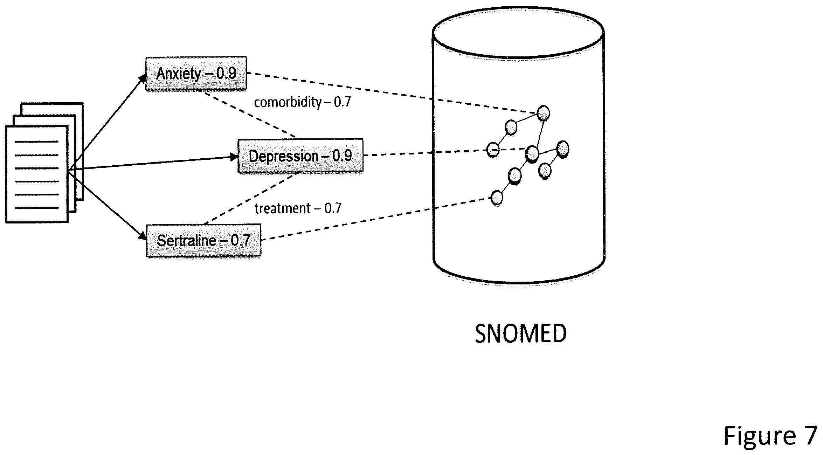

Figure 7 describes the component interaction. In this particular example the component is able to identify the relation between *Anxiety* and *Depression* as *comorbidity* with a score of 0.7, and the relation between *Depression* and *Sertraline* as *treatment,* because the drug prescription for depression is in some cases sertraline.

The labels are available due to previous annotation of SNOMED with the risks, risk factors and treatments, using the terms collector or another module. For example, a link between two risks is labelled with "co-morbidity", a link between a risk and a risk factor is labelled with "risk factor" and a link between a treatment and a risk or risk factor is labelled "treatment".

### Knowledge Graph curator 70

The final module aims at integrating the extracted entities along with their relations, including the scores information and the provenance information into a risk knowledge graph. This provenance information will include the associated document id that supports the relation identification.

The system presents the Risk Knowledge Graph to the clinicians in a very intuitive way, and they can then manually curate and fix some potential inconsistencies of the generated graph.

Embodiments of the invention provide a mechanism that allows creation of a risk knowledge graph 80, with the support of clinicians, which is a foundation to identify patient risks in a more accurate way. The graph may be stored in the same location as the engine, or provided separately. The engine and/or graph may be provided on the cloud.

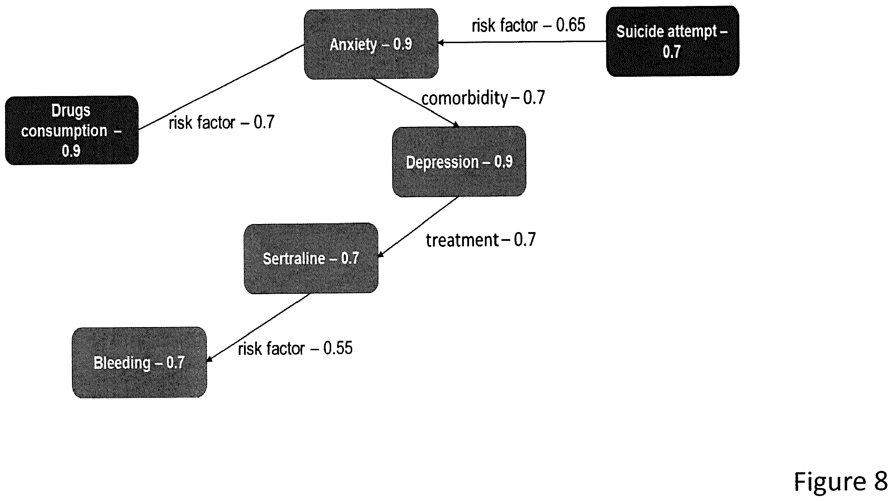

Figure 8 is an illustration of a small part of the graph, showing risks (shaded dark grey), risk factors and treatments. Each entity has a score (.e. Anxiety - 0.9) showing its similarity to the sum of the documents in the retrieved set of documents. 1 indicates an identical term in all the relevant documents. The links are labelled with the relationship between the entities as explained above and the scored using co-occurrence in the set of documents and/or based on their context.

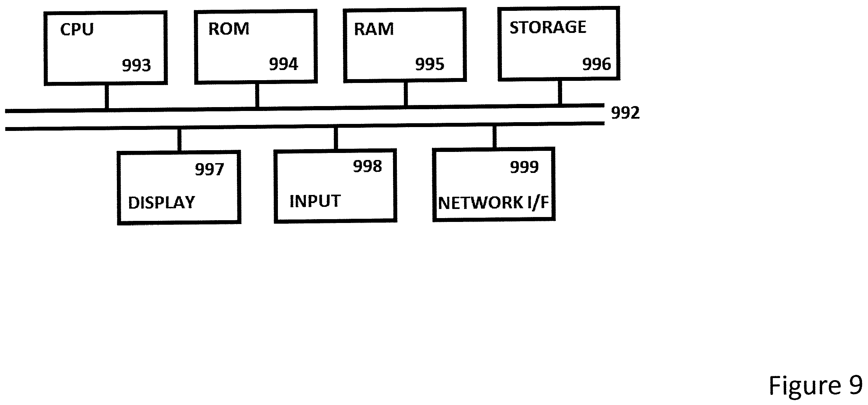

FIGURE 9 is a block diagram of a computing device, such as a data storage server, which embodies the present invention, and which may be used to implement a method of an embodiment. The computing device comprises a computer processing unit (CPU) 993, memory, such as Random Access Memory (RAM) 995, and storage, such as a hard disk, 996. Optionally, the computing device also includes a network interface 999 for communication with other such computing devices of embodiments. For example, an embodiment may be composed of a network of such computing devices. Optionally, the computing device also includes Read Only Memory 994, one or more input mechanisms such as keyboard and mouse 998, and a display unit such as one or more monitors 997. The components are connectable to one another via a bus 992.

The CPU 993 is configured to control the computing device and execute processing operations. The RAM 995 stores data being read and written by the CPU 993. The storage unit 996 may be, for example, a non-volatile storage unit, and is configured to store data.

The display unit 997 displays a representation of data stored by the computing device and displays a cursor and dialog boxes and screens enabling interaction between a user/clinician and the programs and data stored on the computing device. The input mechanisms 998 enable a user/clinician to input data and instructions to the computing device.

The network interface (network I/F) 999 is connected to a network, such as the Internet, and is connectable to other such computing devices via the network. The network I/F 999 controls data input/output from/to other apparatus via the network. Other peripheral devices such as microphone, speakers, printer, power supply unit, fan, case, scanner, trackerball etc may be included in the computing device.

Methods embodying the present invention may be carried out on a computing device such as that illustrated in Figure 9. Such a computing device need not have every component illustrated in Figure 9, and may be composed of a subset of those components. A method embodying the present invention may be carried out by a single computing device in communication with one or more data storage servers via a network. The computing device may be a data storage itself storing at least a portion of the data graph. A method embodying the present invention may be carried out by a plurality of computing devices operating in cooperation with one another. One or more of the plurality of computing devices may be a data storage server storing at least a portion of the data graph.

## Claims

1. A healthcare risks extraction system comprising:
a risk related terms collector to accept input of terms by a clinician, the clinician's terms including terms related to risks in the form of potential diseases, terms related to risk factors that increase the likelihood of disease and terms related to treatments of a medical condition;
a medical entity reconciliator, to standardise and expand the clinicians' terms to include synonyms and equivalent terms using a standardised vocabulary of terms;
a topic detector and tagger, to retrieve a set of documents linked to the expanded terms from a medical document database;
a named entity recognition, resolution and disambiguation, NERD, module to extract entities from the set of document and each aligned to the standardised vocabulary; and
a relation extractor to score relations between the entities based on the co-occurrence of two entities in documents in the retrieved set of documents; wherein
the healthcare risks extraction system is arranged to generate a risk knowledge graph storing the entities and their scored relations.

2. A system according to claim 1, further comprising a knowledge graph curator, to display the risk knowledge graph and to accept clinician input to manually curate the generated graph.

3. A system according to claim 1 or 2, wherein the risk related terms collector is arranged to accept the terms as a list of terms per category of risk, risk factor and treatment.

4. A system according to any of the preceding claims, wherein the topic detector and tagger is arranged to take into account the provenance of the documents.

5. A system according to any of the preceding claims, wherein the risk knowledge graph also stores the provenance of the entities.

6. A system according to any of the preceding claims, wherein the risk related terms collector is arranged to accept annotations by the clinician of the standardised vocabulary of terms, the annotations labelling vocabulary in categories of risks, risk factors and treatments.

7. A system according to any of the preceding claims, wherein the topic detector and tagger is arranged to tag the documents according to categories of risks, risk factors and treatments.

8. A system according to any of the preceding claims, wherein the NERD module scores each entity to reflect the accuracy of a match between the standardised vocabulary term and the corresponding terms in the retrieved linked documents.

9. A system according to any of the preceding claims, further comprising a user input to accept input of terms by a user and a subgraph selection module to select a relevant part of the graph for display to the user.

10. A system according to any of the preceding claims, further comprising a translation module to accept a term in one language and translate it into the equivalent in the language of the standardised vocabulary.

11. A computer-implemented healthcare risks extraction method comprising:
accepting input of terms by a clinician, the clinician's terms including terms related to risks in the form of potential diseases, terms related to risk factors that increase the likelihood of disease and terms related to treatments of a medical condition;
standardising and expanding the clinicians' terms to include synonyms and equivalent terms using a standardised vocabulary of terms;
retrieving a set of documents linked to the expanded terms from a medical document database;
extracting entities from the set of document each aligned to the standardised vocabulary;
scoring relations between the entities based on the co-occurrence of two entities in documents in the retrieved set of documents; wherein
a risk knowledge graph storing the entities and their scored relations is generated.

12. A computer program which when executed on a computer carries out a method according to claim 11.
